# EUROPEAN PATENT SPECIFICATION

(11) **EP 3 678 496 B1**
(45) Date of publication and mention of the grant of the patent: **17.08.2022**
(21) Application number: 18789270.8
(22) Date of filing: 07.09.2018
(51) Int. Cl.: A23L 29/00, A61K 38/46, A61M 5/142, A61K 9/00, C12N 9/20, C12N 9/26, C12N 9/50, A23L 33/00

(54) **DEVICES AND METHODS FOR PREPARING AND ADMINISTERING A NUTRITIONAL FORMULA**
VORRICHTUNGEN UND VERFAHREN ZUR HERSTELLUNG UND VERABREICHUNG EINER ERNÄHRUNGSFORMEL
DISPOSITIFS ET PROCÉDÉS POUR PRÉPARER ET ADMINISTRER UNE FORMULE NUTRITIONNELLE

(30) Priority: 08.09.2017 US 201762555876 P; 06.09.2018 US 201816123629
(43) Date of publication of application: 15.07.2020
(60) Divisional application: 22181695.2
(73) Proprietor: Alcresta Therapeutics, Inc., Newton, MA 02462 (US)
(72) Inventor: FIRST, Eric, Morristown NJ 07960 (US); WIDOM, David, Long Valley NJ 07853 (US); STONE, Albert, Archie, Fuquay-Varina NC 27526 (US); SCHOEVAART, Willem, Robert, Klass, 2623PG Delft (NL); VAN VLIET, Michiel Christian, Alexander, 2613AG Delft (NL)
(74) Representative: Mathys & Squire
(86) International application number: PCT/US2018/049852
(87) International publication number: WO 2019/051162

(56) References cited:
- WO-A1-2013/123139
- WO-A1-2017/066372
- WO-A2-2015/020943
- XIANGHE MENG ET AL: "Preparation and properties of phytosterols with hydroxypropyl [beta]-cyclodextrin inclusion complexes", EUROPEAN FOOD RESEARCH AND TECHNOLOGY, SPRINGER, BERLIN HEIDELBERG, vol. 235, no. 6, 28 September 2012 (2012-09-28), pages 1039-1047, XP035142097, ISSN: 1438-2385, DOI: 10.1007/S00217-012-1833-5

## Description

### FIELD OF THE DISCLOSURE

Embodiments of the present disclosure are directed to devices and methods for preparing and administering a nutritional formula, and, more particularly, to devices and methods for parenteral administration of hydrolyzed lipids, processing phytosterols in nutritional formulas, and/or administering nutritional formulas (e.g., lipid emulsions) from which some or all phytosterols have been extracted.

### BACKGROUND

Premature infants may be born with an immature gastrointestinal (GI) system. As a result, such infants may require specific forms of nutrients, which may be provided in one or more nutritional formulas, so that they receive proper nutrition.

Premature infants are often given parenteral nutrition (PN) within hours of birth. Small amounts of PN may be administered while beginning to prime an infant's GI system. As infants begin to tolerate larger volumes, they may be weaned off of PN and transitioned to oral or enteral feeds, which generally consist of one or more nutritional formulas, including, e.g., mother's own milk (MoM), donor mother's milk (DM), infant formula (IF), additional nutritional fortifiers, and/or a combination of these.

Some infants, however, may not be able to consume formula enterally due to immature, incomplete, or malformed GI systems, surgical procedures, GI infection or inflammation, Crohn's disease, twisting of the intestine, or other causes. As a result, infants may require nutrition provided parenterally or may require a nutritional formula to be supplemented and provided parenterally for more prolonged periods of time.

Additionally, many adults may require parenteral nutrition, such as, for example, those with ulcerative colitis, bowel obstruction, GI infection, inflammation, or abnormalities, Crohn's disease, surgical procedures (e.g., post-operative ileus), short bowel syndrome caused by surgeries, mucositis, graft-versus-host disease, stoma, dysmotility syndrome, or any other medical impairments causing a less-than-fully-functioning GI tract or requiring bowel rest.

Intravenous fat emulsions (IVFEs) have been used for providing nutritional support for PN-dependent individuals, such as adult subjects incapable of receiving enteral nutritional formulas and premature infants. In particular, IVFEs for PN-dependent patients are an important source of essential fatty acids (EFAs) and may be necessary to prevent EFA deficiency. Soybean oil (SO)-based IVFEs have been common IVFEs available on the market in the United States. Examples of IVFEs on the market include Liposyn^{®} II, Liposyn^{®} III, Lipofundin^{®} MCT, Lipofundin^{®} N, Structolipid^{®}, Intralipid^{®}, Ivelip^{®}, ClinOleic^{®}, and SMOF^{®}. Other IFVEs are based on palm or coconut oil, olive oil, and egg yolk phospholipids. Use of these emulsions in PN feedings in neonatal intensive care units is generally not recommended, but does occur.

PN carries with it the risk of progressive liver disease for premature infants, children, and adults alike. Data shows that components of parenteral SO are associated with the pathogenesis of PN-associated cholestasis (PNAC) and parenteral nutrition-associated liver dysfunction (PNALD) in people receiving PN. PNALD in particular has been associated with the use of greater than 1g/kg/day of SO-based IVFEs as part of PN prescriptions. As a result, lipid emulsions for PN that use plant-based oil include a black box warning indicating that their use can lead to PNALD, which may result in death. Black box warnings for PNALD on lipid emulsions products have been in place for decades and the risks associated with PNALD have been known since at least 1980 (e.g., see LEVENE MI, WIGGLESWORTH JS, DESAI R: PULMONARY FAT ACCUMULATION AFTER INTRALIPID® INFUSION IN THE PRETERM INFANT. LANCET 1980; 2(8199):815-8). The exact etiology of PNALD is unclear, but likely results from multifactorial etiologies that include high intake of lipids, high doses of linoleic acid (found in SO), high intake of phytosterols, extremely short GI tracts, lack of use of enteral nutrition, disruption of the enterohepatic circulation, and/or recurrent sepsis. Specifically, evidence suggests an association between high plasma phytosterol concentrations in SO-based lipid emulsions and the onset and severity of PNAC and PNALD. During parenteral administration of lipid emulsions in particular, plasma levels of phytosterols may be many fold higher than during enteral administration.

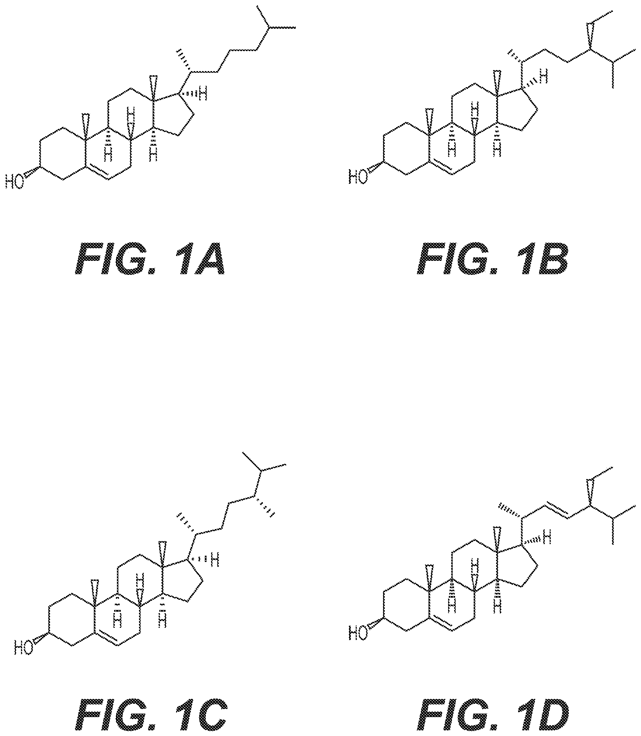

Phytosterols, or plant sterols, are a family of molecules found in the cell membranes of plants, where they play important roles similar to cholesterol in humans. The most common phytosterols in the human diet are campesterol, sitosterol, and stigmasterol. They are structurally similar to cholesterol, except that they contain some substitutions at the C24 position on the sterol side chain. FIG. 1A depicts the chemical composition of cholesterol. FIG. 1B depicts sitosterol, FIG. 1C depicts campesterol, and FIG. 1D depicts stigmasterol.

Phytosterol accumulation in the body, associated with the use of IVFEs, may be caused by the bypass of the protective mechanisms in the GI system to which enteral nutrition is subjected. Under enteral feeding conditions, phytosterol elimination occurs by intestinal and hepatic ABCG5/G8 transporters. Dietary phytosterols are excreted into the intestinal lumen by intestinal ABCG5/G8 transporters, preventing the entry into the bloodstream of more than 95% of the ingested phytosterols. The small amounts of phytosterols absorbed by the gut are then excreted by the hepatic ABCG5/G8 transporters into bile produced by the body. During PN administration of lipid emulsions containing phytosterols, however, the direct entry of phytosterols into the bloodstream may lead to much higher levels of phytosterols in the blood than are typically achievable when people receive nutrients enterally. This can lead to toxic accumulation of phytosterols in the liver due to the limits of hepatic excretion. Currently available strategies to overcome this issue include reducing the dose of administered parenteral soybean oil and/or the replacement of parenteral soybean oil with alternative parenteral lipid emulsions. Fish-based IVFEs may also be used, or may be used in combination with plant-based oils, because fish-oils do not contain phytosterols, and thus use of fish-oil may cut down on the amount of phytosterols delivered to a subject.

In an attempt to lower the use of soybean oil, lipid emulsions have been developed in which soybean oil is replaced with coconut or palm oil. Commercially available preparations of this type contain, for example, 50% long-chain triglycerides as soybean oil and 50% medium-chain triglycerides. An alternative lipid preparation is based upon the use of olive oil and soybean oil. Olive oil is rich in oleic acid and low in omega-6 long-chain polyunsaturated acids. Table 1 below summarizes sterol compositions of IVFEs.

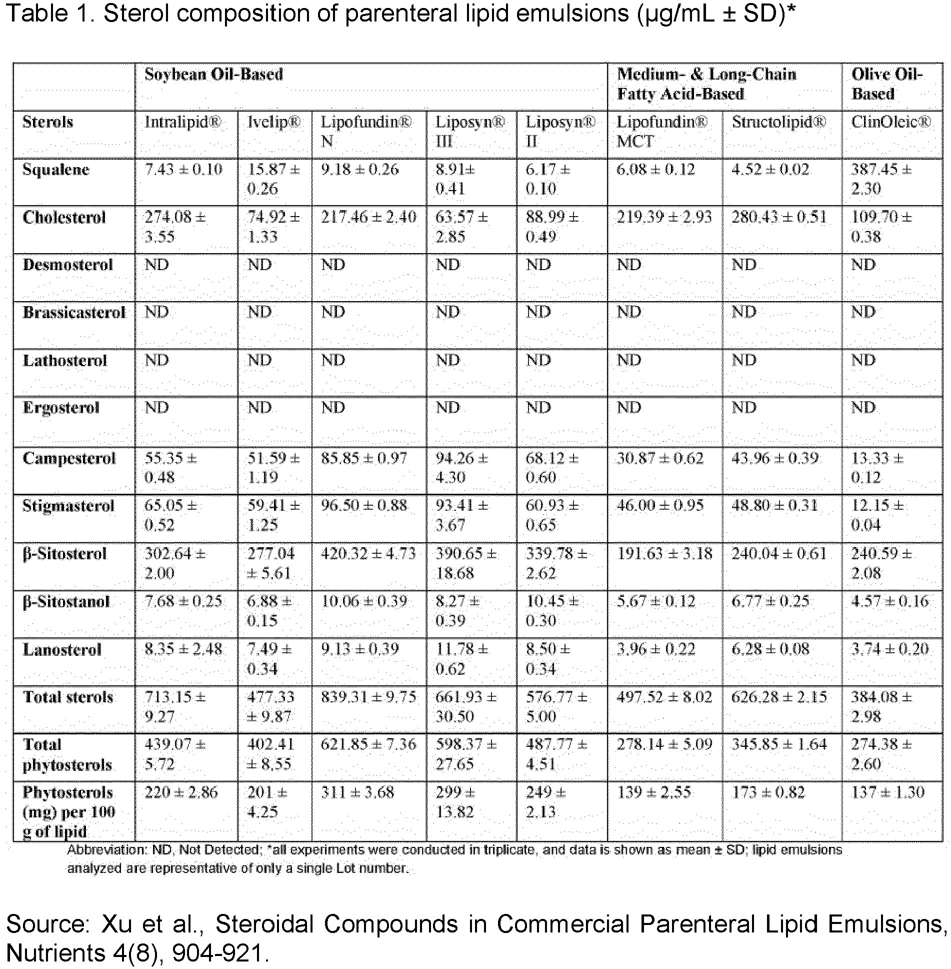

Long-chain fatty acids are important to human health and development. Many long-chain fatty acids are consumed as triglycerides, in which three long-chain fatty acids are bound to a glycerol molecule via ester linkages. Absorption of long-chain triglycerides (LCTs) by the body first requires the enzymatic action of lipases (e.g. pancreatic lipase) and bile salts, which digest triglycerides through hydrolysis, breaking them down into a monoglyceride and two free fatty acids. Digestion products consisting of a mixture of tri-, di-, and monoglycerides and free fatty acids, which, together with the other fat soluble contents of the diet (e.g. the fat soluble vitamins and cholesterol) and bile salts, form mixed micelles in the watery duodenal contents. Once broken down, the monoglycerides and free fatty acids may be absorbed by enterocytes-epithelial cells lining the small intestine-for example, in the region of the jejunum. The contents of these micelles (but not the bile salts) enter the enterocytes, where they are resynthesized into triglycerides and packaged into chylomicrons, which are released into the lacteals (the capillaries of the lymph system of the intestines). Medium-chain triglycerides (MCTs) are absorbed directly into the bloodstream.

Exocrine pancreatic function may not be fully developed at birth in premature infants, and so premature infants may lack sufficient quantities of the enzyme lipase, which is necessary to break down triglycerides. At birth, the mother provides an "on-board lipase," called bile salt-stimulated lipase (BSSL), also known as carboxyl ester lipase or bile salt-dependent lipase, which is provided to the infant through breast milk. While this may partially compensate for poor endogenous production, BSSL production may be insufficient for supporting proper fat absorption. Additionally, the majority of fats in mother's milk are in the form of palmitic acid (n-16), which is an MCT, and thus mother's milk may lack sufficient LCTs, e.g., those containing docosahexaenoic acid (DHA, 22:6 n-3) and arachidonic acid (ARA 20:4 n-6), which are critical in membrane structure, function, and neuronal, retinal, and other tissue development. In donor milk, during the pasteurization process, lipase that was present may be inactivated by exposure to high heat, and thus LCT fats are not as readily broken down. As a result, an infant may suffer from feeding intolerance due to the inability to absorb these larger LCTs, irritating the gut mucosa and initiating localized inflammation. The ability to more efficiently process and absorb LCTs may lead to better overall nutrient absorption and thus growth.

For at least the above reasons, people suffering from various malabsorption impairments may not be able to receive nutritional formula enterally, for prolonged periods of time. Additionally, people suffering from such impairments may be unable to adequately digest LCTs and other forms of fat through hydrolysis, inhibiting absorption of the fatty acids required to maintain health. Exemplary impairments for infant, child, or adult subjects include, but are not limited to, the following: compromised pancreatic output, acute and chronic pancreatitis, pancreatic cancer, pancreatic insufficiency, cystic fibrosis, cerebral palsy, Crohn's disease, irritable bowel syndrome, chronically abnormal epithelium, amyloidosis, celiac disease, ischemia, radiation enteritis, tropical sprue, Whipple disease, inadequate gastric mixing, rapid emptying, or both, Billroth II gastrectomy, gastrocolic fistula, gastroenterostomy, insufficient digestive agents, biliary obstruction and cholestasis, cirrhosis, chronic pancreatitis, cholestyramine-induced bile acid loss, cystic fibrosis, lactase deficiency, pancreatic cancer, pancreatic resection, sucrase-isomaltase deficiency, abnormal milieu, abnormal motility secondary to diabetes, scleroderma, hypothyroidism, or hyperthyroidism, bacterial overgrowth due to blind loops (deconjugation of bile salts), diverticula in the small intestine, Zollinger-Ellison syndrome (low duodenal pH), acutely abnormal epithelium, acute intestinal infections, alcohol, neomycin, impaired transport, abetalipoproteinemia, Addison disease, blocked lacteals due to lymphoma or tuberculosis, intrinsic factor deficiency (as in pernicious anemia), lymphangiectasia, jejunoileal bypass for obesity, short bowel syndrome, intestinal failure, gastroschisis, secondary to HIV or burns, or other conditions. Furthermore, patients requiring PN may be at increased risk for complications associated with phytosterols in nutritional formulas. Other people may need or want additional dietary supplementation. Improvements are required to address these and other issues.

WO 2017/066372 A1 discloses an enteral feeding device for hydrolyzing triglycerides in a nutritional formula. The device may include a body housing a chamber, an inlet configured to fluidly couple with a source of nutritional formula, and an outlet configured to fluidly couple with an enteral feeding tube. The device may include a headspace and a plurality of particles contained within the chamber, wherein the lipase is covalently bonded to the plurality of particles. The device may include an inlet filter located between the inlet and the chamber, wherein the inlet filter contains a first plurality of openings, and an outlet filter located between the chamber and the outlet, wherein the outlet filter has a second plurality of openings smaller than the plurality of particles.

### SUMMARY

The invention is defined in independent claim 1. Exemplary embodiments of the disclosure may be drawn to a device having one or more chambers. The one or more chambers may contain immobilized lipase and a phytosterol processing excipient. The device may also include an inlet fluidly connected to one of the one or more chambers, wherein the inlet is configured to receive nutritional formula into one of the one or more chambers. The device may further include an outlet through which nutritional formula is configured to flow after passing through the one or more chambers.

Various embodiments of the device may include one or more of the following features: the one or more chambers may comprise at least two chambers, and the at least two chambers may be fluidly connected to one another; the immobilized lipase may be contained within a first of the at least two chambers, and the phytosterol processing excipient may be contained within a second of the at least two chambers; a third chamber may be fluidly connected to the second chamber; the phytosterol processing excipient may be one of a cyclodextrin or a phytosterol metabolizing enzyme; the one or more chambers may comprise one chamber, and the immobilized lipase and the phytosterol processing excipient may both be contained within the one chamber; the one or more chambers may comprise a first chamber and a second chamber, and the immobilized lipase and the phytosterol processing excipient may both be contained within the first chamber, and the second chamber may be a phytosterol collection reservoir; and a connector may be coupled to the outlet, and the connector may be configured to connect the outlet to one of a parenteral feeding attachment or an enteral feeding attachment.

In other exemplary embodiments, a device may include a first chamber configured to fluidly connect to a source of nutritional formula and a second chamber fluidly connected to the first chamber. The device may also include immobilized lipase contained within the first chamber and positioned within a flow path along which the nutritional formula flows when received within the first chamber, and a phytosterol processing excipient contained within the second chamber and positioned within a flow path along which the nutritional formula flows when received within the second chamber. The device may further include an outlet through which the nutritional formula is configured to flow after passing through the first chamber and the second chamber.

Various embodiments of the device may include one or more of the following features: the outlet may be configured to be connected to a parenteral feeding attachment or an enteral feeding attachment; a third chamber may be fluidly connected to the second chamber; and the phytosterol processing excipient may be one of a cyclodextrin or a phytosterol metabolizing enzyme.

In other exemplary embodiments, a device may include a chamber, an inlet configured to receive a nutritional formula into the chamber, an outlet configured to allow the nutritional formula to exit the chamber, and a phytosterol processing excipient contained within the chamber.

Various embodiments of the device may include one or more of the following features: the phytosterol processing excipient may be one of a cyclodextrin or a phytosterol metabolizing enzyme; the phytosterol processing excipient may be an enzyme configured to bioconvert a phytosterol into a cholesterol; at least one of an antioxidant or an immobilized lipase may be contained within the chamber; a parenteral feeding attachment or an enteral feeding attachment may be fluidly connected to the chamber via one or more conduits or connectors; the chamber may be a first chamber and the device may further include a second chamber fluidly connected to the first chamber; the second chamber may be a phytosterol collection reservoir; and the second chamber may contain immobilized lipase.

In other exemplary embodiments, a method of removing phytosterol may include mixing cyclodextrin with a nutritional formula containing the phytosterol, allowing the cyclodextrin to bind with the phytosterol to form a cyclodextrin-phytosterol conjugate, and removing the cyclodextrin-phytosterol conjugate from the nutritional formula.

Various embodiments of the device may include one or more of the following features: the cyclodextrin may include free cyclodextrin, and the method may further comprise (i) adding free cyclodextrin to the nutritional formula prior to mixing the cyclodextrin with the nutritional formula and (ii) precipitating out the cyclodextrin-phytosterol conjugate prior to removing the cyclodextrin-phytosterol conjugate; the cyclodextrin may include cyclodextrin immobilized to a solid matrix, and the method may further comprise adding the immobilized cyclodextrin to the nutritional formula prior to mixing the cyclodextrin with the nutritional formula; removing the cyclodextrin-phytosterol conjugate from the nutritional formula may include removing the solid matrix from the nutritional formula; at least one of mixing the cyclodextrin with the nutritional formula containing phytosterol or allowing the cyclodextrin to bind with the phytosterol may include agitating the nutritional formula and/or heating the nutritional formula; separating the phytosterol from the nutritional formula may include at least one of filtering, centrifugation, or elutriation; and mixing the cyclodextrin with the nutritional formula containing phytosterol may include creating a solution of supersaturated cyclodextrins.

Both the foregoing general description and the following detailed description are exemplary and explanatory only and are not restrictive of the features, as claimed. As used herein, the terms "comprises," "comprising," "includes," or other variations thereof, are intended to cover a non-exclusive inclusion such that a process, method, article, or apparatus that comprises a list of elements does not include only those elements, but may include other elements not expressly listed or inherent to such a process, method, article, or apparatus. Additionally, the term "exemplary" is used herein in the sense of "example," rather than "ideal." It should be noted that all numeric values disclosed or claimed herein (including all disclosed values, limits, and ranges) may have a variation of +/- 10% (unless a different variation is specified) from the disclosed numeric value. Moreover, in the claims, values, limits, and/or ranges means the value, limit, and/or range +/-10%. Further, although some embodiments are discussed in terms of use for infants, it is contemplated that embodiments of the device may be used for subjects of all ages, including the elderly, adults, children, and infants.

### BRIEF DESCRIPTION OF THE DRAWINGS

The accompanying drawings, which are incorporated in and constitute a part of this specification, illustrate the disclosed embodiments, and together with the description, serve to explain the principles of the disclosed embodiments. There are many aspects and embodiments described herein. Those of ordinary skill in the art will readily recognize that the features of a particular aspect or embodiment may be used in conjunction with the features of any or all of the other aspects or embodiments described in this disclosure. In the drawings:
Fig. 1A illustrates the chemical structure of cholesterol.
Fig. 1B illustrates the chemical structure of sitosterol.
Fig. 1C illustrates the chemical structure of campesterol.
FIG. 1D illustrates the chemical structure of stigmasterol.
FIG. 2 illustrates, in schematic form, an exemplary device for processing a nutritional formula, according to embodiments of the present disclosure.
FIG. 3A illustrates the chemical structure of α-cydodextrin.
FIG. 3B illustrates the chemical structure of β-cyclodextrin.
FIG. 3C illustrates the chemical structure of γ-cyclodextrin.
Fig. 4 schematically illustrates bioconversion of mevalonic acid into sitosterol and cholesterol via the metabolic systems of various organisms.
Fig. 5 is a flow chart depicting an exemplary method of preparing and administering a nutritional formula, according to embodiments of the present disclosure.
FIG. 6A illustrates a mixing tank in which free cyclodextrins are binding to free phytosterols, according to embodiments of the present disclosure.
FIG. 6B illustrates the removal of the bound cyclodextrin-phytosterol conjugates from the mixing tank of FIG. 6A via filtration.
FIG. 6C illustrates the removal of the bound cyclodextrin-phytosterol conjugates from the mixing tank of FIG. 6A via centrifugation.
FIG. 7 is a flow chart depicting an exemplary method of preparing bulk nutritional formula, according to embodiments of the present disclosure.

### DETAILED DESCRIPTION

Reference will now be made in detail to the exemplary embodiments of the present disclosure described below and illustrated in the accompanying drawings. Wherever possible, the same reference numbers will be used throughout the drawings to refer to same or like parts.

Additional objects and advantages of the embodiments will be set forth in part in the description that follows, and in part will be obvious from the description, or may be learned by practice of the embodiments. It is to be understood that both the foregoing general description and the following detailed description are exemplary and explanatory only and are not restrictive of the claims.

Aspects of the present disclosure are described with reference to devices and methods for hydrolyzing lipids in nutritional formulas (e.g., lipid emulsions for parenteral nutrition), devices and methods for processing (e.g., extracting or converting) phytosterols in nutritional formulas, and devices for administering such nutritional formulas parenterally or enterally. While embodiments of the present disclosure may be described in reference to parenteral nutrition (PN) formulas, it will be understood that embodiments of the present disclosure may be applicable to a variety of nutritional formulas (e.g., enteral nutritional formulas, mother's milk, donor milk, any type of supplemented milk, etc.).

As used herein, the term "nutritional formula" may include complex mixtures containing, for example, proteins, carbohydrates, fats, water, minerals, and/or vitamins. This may include liquid foods that are specially formulated and processed; liquids used for the partial or exclusive feeding of a person by means of oral intake or feeding by tube (either parenterally or enterally); liquids used for the dietary management of a person who, because of therapeutic or medical need, has limited or impaired capacity to ingest, digest, absorb, or metabolize ordinary foodstuffs or certain nutrients; liquids that meet medically determined nutrient requirements; and liquids designed to deliver to a subject nutrients that cannot be provided to the subject via dietary management and modification of the normal diet alone.

In some embodiments, nutritional formulas may be delivered to a subject under medical supervision, may be intended only for a person receiving active and ongoing medical supervision, or may be delivered to the subject for home use, either when supervised or unsupervised. Nutritional formulas may be packaged as a dry powder and then mixed with a solvent to form a solution or may be packaged as a liquid nutritional formula, beverage, or drink. In some embodiments, a nutritional formula may be commercially available, or may be prepared by a healthcare professional before feeding. In some embodiments, a nutritional formula may include at least one medicament prescribed for the subject in need of the medicament and/or nutritional formula, or the nutritional formula may itself be the prescribed medicament. A nutritional formula may be an infant and/or toddler formula as a complete or partial substitute for human milk, may be donor milk, or mother's milk (infant's own mother or other mother's milk), whether pasteurized or unpasteurized.

A nutritional formula may or may not include at least one fat in triglyceride form, such as short-chain triglycerides (SCTs), MCTs, and LCTs. A nutritional formula may include an intravenous fat/lipid emulsion (IVFE). In some embodiments, a nutritional formula may further include at least one nutrient selected from water, maltodextrin, protein, hydrolyzed protein, amino acids, peptides, SCTs, MCTs, diglycerides, monoglycerides, cornstarch, fish oil, soybean oil, rapeseed oil, cottonseed oil, sunflower oil, olive oil (oils may or may not be refined), soluble fiber, lecithin, magnesium chloride, sodium ascorbate, guar gum, calcium phosphate, salt, choline chloride, phosphoric acid, calcium citrate, sodium phosphate, taurine, magnesium oxide, zinc sulfate, potassium chloride, niacinamide, ferrous sulfate, calcium pantothenate, manganese sulfate, pyridoxine hydrochloride, copper sulfate, thiamine mononitrate, beta-carotene, riboflavin, vitamin a palmitate, folic acid, biotin, sodium selenate, chromium chloride, potassium iodide, sodium molybdate, soluble fiber, fructooligosaccharide, probiotic, citric acid, vitamin A, vitamin D, vitamin E, vitamin B_{1,} vitamin B_{2,} vitamin B_{3,} vitamin B_{5,} vitamin B_{6,} vitamin B_{7,} vitamin B_{9,} and vitamin B₁₂. Exemplary nutritional formulas and systems are described in U.S. Patent Application No. 14/378,856, filed August 14, 2014, now U.S. Patent No. 9,668,942.

As used herein, the term "parenteral nutrition formula," "PN formula," or "PN" may include any nutritional formula intended for parenteral administration.

In some aspects, exemplary PN formulas may be, contain, or be supplemented with, IVFEs based on plant oils, such as soybean oil, rapeseed oil, cottonseed oil, sunflower oil, olive oil, fish oil, krill oil, combinations of both vegetable and non-vegetable-based oils, and the one or more oils may or may not be refined. Such plant oils may include concentrations of phytosterols, such as sitosterol, campesterol, or stigmasterol, which are depicted in FIGS. 1B, 1C, and 1D, respectively.

As used herein, the term "processing" with respect to any nutritional formula or PN formula may refer to altering the formula in one or more of a variety of ways prior to administering the formula to a subject. "Processing" may refer, for example, to the hydrolysis of lipids within a nutritional formula, the removal of phytosterols from a nutritional formula, the conversion of phytosterols to cholesterols or other metabolites in a nutritional formula, or a combination thereof. "Processing" may also refer to other alterations to a nutritional formula, such as supplementing the formula.

Some embodiments of the present disclosure may be drawn to devices and methods for parenterally administering nutritional formulas having hydrolyzed triglycerides (e.g., into monoglycerides and free fatty acids) to subjects unable to receive nutritional formulas enterally, so as to, e.g., increase concentrations of fatty acids that can be absorbed by such subjects, and to devices and methods for reducing the amount and/or concentration of phytosterols in nutritional formulas for parenteral or enteral administration. Embodiments of the present disclosure are thus drawn to devices and methods for, e.g., increasing the amount of absorbable nutrients in parenteral nutritional formulas, delivering such nutritional formulas parenterally, delivering such nutritional formulas enterally, and/or decreasing concentrations of phytosterols in nutritional formulas so as to decrease the risk of liver disorders, such as PNALD, PNAC, and other disorders.

Exemplary devices may include a vessel for receiving a nutritional formula, a chamber containing immobilized lipase through which the nutritional formula may be passed in order to hydrolyze lipids in the nutritional formula, and a parenteral feeding system for delivering the nutritional formula parenterally. In some embodiments, the nutritional formula may be a parenteral formula, and in some embodiments, the nutritional formula may be a lipid source intended to supplement a separate source of nutritional formula.

Further exemplary devices may include a phytosterol processing chamber through which a nutritional formula may pass in order to convert or remove phytosterols in the nutritional formula. In some embodiments, exemplary devices may be fluidly connected to a separate source of nutritional formula and/or to a parenteral or enteral feeding system for delivering a nutritional formula to a subject. Exemplary devices may be used to deliver a nutritional formula parenterally or enterally.

In some embodiments, exemplary devices may include both a chamber containing immobilized lipase and a phytosterol processing chamber, such that a nutritional formula may pass through both chambers. In further embodiments of exemplary devices, a chamber containing immobilized lipase may also be a phytosterol processing chamber, such that passing a nutritional formula through the chamber both hydrolyzes triglycerides and removes or converts phytosterols in the nutritional formula. Exemplary devices and exemplary systems in which they may be included are described further below.

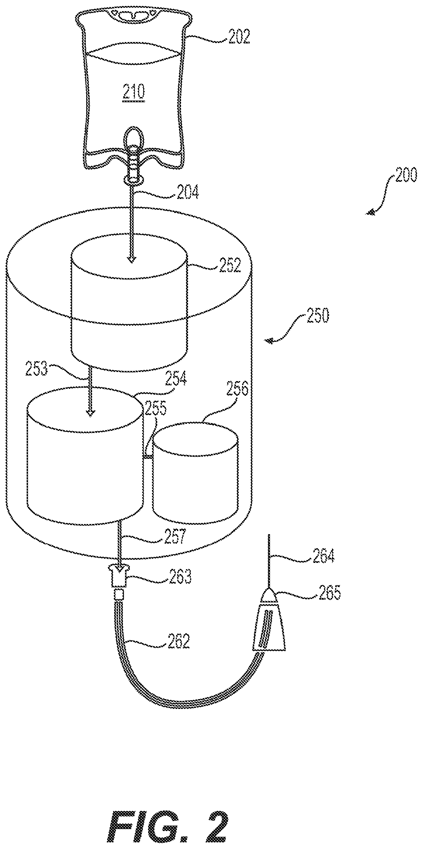

FIG. 2 illustrates an exemplary feeding system 200 for providing a nutritional formula 210 to a subject, e.g., via an intravenous tube. While system 200 is described herein as having a given configuration and components, it will be apparent to those of ordinary skill in the art that variations of system 200 are also possible. For example, any parts of system 200 may be arranged in a different configuration or order, or may be omitted completely.

System 200 may include a source 202 of nutritional formula 210 fluidly connected to a formula processing device 250 via a conduit 204. Formula processing device 250 may be fluidly connected to a conduit 262 via a connector 263. Conduit 262 may be fluidly connected (e.g., removably connected) to, e.g., a needle 264 via a connector 265. Needle 264 and/or conduit 262 may be configured for administration to a subject. In some embodiments conduit 262 may be directly connected to needle 264, without including a connector 265 and/or may be directly connected to formula processing device 250, without a connector 263. In some embodiments, formula processing device 250 may be a point-of-care device or a bulk processing device.

Formula processing device 250 may include one or more of chambers 252, 254, 256 for processing nutritional formula 210 flowing from source 202. A hydrolysis chamber 252 may be configured to hydrolyze lipids in nutritional formula 210 as nutritional formula 210 flows from tube 204. A phytosterol processing chamber 254 may be fluidly connected to hydrolysis chamber 252 via a conduit 253. Phytosterol processing chamber 254 may also be fluidly connected to a phytosterol collection reservoir 256 via a conduit 255. An exit conduit 257 may connect phytosterol processing chamber 254 to an egress from formula processing device 250, where connector 263 may connect exit conduit 257 of formula processing device 250 to conduit 262, through which nutritional formula 210 may flow to a subject.

System 200 may be a parenteral feeding system. In some embodiments, some or all parts of system 200 may be approved for medical use by, e.g., a government regulatory agency. In some embodiments, parts of system 200 may be detachable from one another; for example, source 202 may be detachable from conduit 204, conduit 204 may be detachable from formula processing device 250, formula processing device 250 may be detachable from conduit 262, and conduit 262 may be detachable from needle 264. In some embodiments, system 200 may be configured to allow for nutritional formula 210 to pass through system 200 from source 202 through conduit 262 and, for example, into a subject.

In some embodiments, system 200 may be equipped with a pump to promote the flow of nutritional formula 210 through system 200, such as a peristaltic pump, an elastomeric pump, a multi-channel pump, a syringe pump, and/or a smart pump. In other embodiments, nutritional formula 210 may be allowed to pass through system 200 under power of gravity, capillary action, or pressure applied to nutritional formula 210 via, for example, an inflatable balloon or a syringe piston. When gravity is used to facilitate the flow of nutritional formula 210, the relative positioning of source 202 of nutritional formula 210 may allow nutritional formula 210 to flow through system 200, under the influence of gravity alone. For example, a source 202 of nutritional formula 210 may be placed above conduit 204, above device 250, and/or above the subject, as shown in FIG. 2. In further embodiments, a combination of pumping, gravity, pressure, and/or capillary action may allow for nutritional formula 210 to pass through system 200.

In some embodiments, some or all parts of system 200 may be sterilized. In some embodiments, system 200 may be configured to provide either parenteral nutrition or enteral nutrition. For example, needle 264 may be replaceable with a tube, port, or other attachment suitable for enteral feeding.

Exemplary embodiments of parts of system 200 and its components may be described in U.S. Patent Application No. 15/291,530, filed October 12, 2016, and U.S. Patent Application No. 14/378,856, filed August 14, 2014, now U.S. Patent No. 9,668,942. In the present disclosure, it is contemplated that system 200 may be suited in particular to hydrolyzing lipids in nutritional formulas, removing phytosterols from, or converting phytosterols within, nutritional formulas, and/or delivering such formulas to subjects.

Source 202 may be any source suitable for supplying a nutritional formula. For example, source 202 may be a vessel such as an intravenous or enteral feeding bag, a vial, a syringe, or any other suitable container containing a nutritional formula. Source 202 may be a single-use source (such as a disposable vessel) or a multi-use source (such as a resealable and/or sterilizable container). Source 202 may or may not be detachable from other elements of system 200. In some embodiments, source 202 may be a sterilized or sterilizable container. In some embodiments, source 202 may be pre-filled with a prepared nutritional formula, such as nutritional formula 210. In some embodiments, source 202 may be provided in a closed, sealed configuration and may be opened shortly prior to administering the nutritional formula 210 within to a subject.

In some embodiments, a user (e.g., healthcare provider, patient, patient guardian, pharmacist, or other user) may attach source 202 to conduit 204 and/or formula processing device 250 prior to use. For example, the user may select a pre-filled source 202 containing a desired nutritional formula 210 and may attach source 202 to conduit 204 for use. In some embodiments, source 202 may be pre-filled, and a user may select between different types of nutritional formulas or combinations of nutritional formulas and/or may select between different volumes of nutritional formulas, depending, e.g., on the needs of the subject. In such embodiments, source 202 may have a sealed opening that is either unsealed prior to attachment to conduit 204 or formula processing device 250, or the action of attaching source 202 to conduit 204 or formula processing device 250 may break the seal (e.g., perforate, puncture, displace, or otherwise open the seal).

In some embodiments, a valve or other mechanical structure may be used to maintain nutritional formula 210 in source 202 prior to use and/or to control the flow of nutritional formula 210 out of source 202 and into formula processing device 250. In still other embodiments, a user may fill source 202 with the desired type of nutritional formula, combination of nutritional formulas, and/or desired volume of nutritional formula prior to and/or during use. In some embodiments, source 202 may be mixed, agitated, heated, cooled, or otherwise primed before and/or during use.

In some embodiments, multiple sources 202 may feed into conduit 204 and/or formula processing device 250. For example, a first source may include a nutritional formula in powder form, and a second source may include liquid to be mixed with the powdered nutritional formula. In further embodiments, a first source may include a liquid nutritional formula, and a second source may include a nutritional formula supplement, such as a fatty acid supplement (e.g., an IVFE).

Nutritional formula 210 may be any nutritional formula or combination of nutritional formulas, as has been described previously herein. In some embodiments, nutritional formula 210 may be in liquid form. In some embodiments, nutritional formula 210 may include triglycerides, phytosterols, or both triglycerides and phytosterols. In some embodiments, nutritional formula 210 may be a PN formula. In some embodiments, nutritional formula 210 may be, or may include, an IVFE. In some embodiments, nutritional formula 210 may be in powdered form, and may be combined with a liquid prior to entering conduit 204.

Nutritional formula 210 may include one or more of, e.g., a short-chain, medium-chain, or long-chain fatty acid, for example, a long-chain polyunsaturated fatty acid ("LC-PUFA") triglyceride. Exemplary fats (e.g., lipids) in nutritional formula 210 may include natural or structured lipids, or omega-3 or omega-6 fatty acids, like docosahexaenoic acid ("DHA"), eicosapentaenoic acid ("EPA"), alpha-linolenic acid ("ALA"), arachidonic acid ("ARA" or "AA"), and/or linoleic acid ("LA"). In some embodiments, nutritional formula 210 may include an oil or oil extract from a plant source, such as one or more of soybean oil, palm or coconut oil, or olive oil, or any suitable oil. In some embodiments, nutritional formula 210 may include an oil or oil extract from a marine source, such as one or more of fish oil or krill oil, or any suitable oil. In some embodiments, nutritional formula 210 may be a lipid source, e.g., configured to supplement another nutritional formula.

Nutritional formula 210 may flow through formula processing device 250 in any suitable manner. In some embodiments, system 200 may gravity-feed nutritional formula 210 through formula processing device 250, where lipids in nutritional formula 210 may be hydrolyzed, and then nutritional formula 210 having hydrolyzed lipids may flow into conduit 262 under the force of gravity. In some embodiments, nutritional formula 210 may be stored under pressure in source 202.

In some embodiments, source 202 or portions of source 202 may be deformable. A user may squeeze source 202, forcing nutritional formula 210 out of source 202 and into formula processing device 250. The source or portions of source 202 may deform as flow evacuates source 202, for example, driven via a pressure differential. In some embodiments, a motorized compression roller or other mechanical device may be included and may compress source 202 in a controlled manner at a given rate or over a given amount of time. In some embodiments, a pump, e.g., a continuous or peristaltic pump (which may be manually operated or electronic), may be included in formula processing device 250 or attached to source 202 to urge nutritional formula 210 out of source 202. In other embodiments, a source of negative pressure may be connected to one or more chambers in formula processing device 250, creating a vacuum into which a flow of nutritional formula 210 may be drawn.

To facilitate the emptying of source 202, source 202 may include a valve or other flow-control device and/or may include an air release to equalize pressure as nutritional formula 210 is emptied from source 210. In some embodiments, source 202 may include measurement lines so that a user may observe how much of nutritional formula 210 has been released into formula processing device 250 and/or how much nutritional formula 210 remains in source 202. In some embodiments, a syringe or other delivery device may feed nutritional formula 210 into source 202 either prior to and/or during use of system 200, and nutritional formula 210 may then flow into formula processing device 250. In some embodiments, a vibrating motor may be included in or attached to formula processing device 250 and/or source 202, to vibrate formula processing device 250 and/or source 202, agitate nutritional formula 210 and/or one or more chambers in formula processing device 250, and/or assist the flow of nutritional formula 210 through the one or more chambers in formula processing device 250.

Conduit 204 may be any conduit suitable for conveying nutritional formula 210 from source 202 to formula processing device 250. Conduit 204 may be, for example, medical-grade tubing. Conduit 204 may connect source 202 and formula processing device 250 using any connector known in the art, such as by a luer-lock connection, threads, projections, grooves, deformable or expandable structures, and/or any other suitable mechanism for connecting elements for carrying nutritional formula 210 from source 202 to device 250. One suitable connector known in the art is the ENFit^{®} connector (GEDSA). In some embodiments, conduit 204 may not be present, and source 202 may connect directly to formula processing device 250 via any suitable connection means.

Formula processing device 250 may be a device containing one or more chambers, pumps, and/or conduits for processing a nutritional formula, such as nutritional formula 210. A combination of chambers and/or conduits in formula processing device 250 may allow for nutritional formula 210 to enter formula processing device 250 via conduit 204 and exit via conduit 257. In some embodiments, formula processing device 250 may be a single device. In other embodiments, formula processing device 250 may include a series of devices, each of which may contain chambers, pumps, and/or conduits. For example, formula processing device 250 may be a single device including a hydrolysis chamber 252 and a phytosterol processing chamber 254 arranged in series and connected by conduits, valves, or directly connected to each other such that nutritional formula 210 may pass through the chambers 252, 254 in series (e.g., first chamber 252 and then chamber 254, or vice versa). In some embodiments, formula processing device 250 may include only one chamber configured to process a nutritional formula in a variety of ways (e.g., by both hydrolyzing fats in the nutritional formula and removing or converting phytosterols). In some embodiments, rather than incorporating different chambers into one device, formula processing device 250 may consist of a series of separate devices that may be arranged in any order or used individually, and each separate device may include its own chamber.

While in Figure 2, hydrolysis chamber 252 is depicted as being "upstream" from phytosterol processing chamber 254, in some embodiments this positioning may be reversed, such that nutritional formula flows from source 202 first into phytosterol processing chamber 254, and then into hydrolysis chamber 252. In some embodiments, formula processing device 250 may include only a hydrolysis chamber 252 or only a phytosterol processing chamber 254. It is to be understood by those of ordinary skill in the art that a variety of configurations for each chamber within formula processing device 250 are possible.

In some embodiments, formula processing device 250 may be openable, such that parts of formula processing device 250 may be removed, replaced, and/or cleaned. In some embodiments, formula processing device 250 may be made entirely or partially of a clear plastic or glass so that elements within formula processing device 250 are visible to a user. In some instances, this may allow the user to ensure proper flow through formula processing device 250, for example, by visual inspection. In other embodiments, formula processing device 250 may be opaque.

In some embodiments, formula processing device 250 may include one or both of an inlet filter and an outlet filter. In some embodiments, each chamber within formula processing device 250 may also or alternatively include one or both of an inlet filter and an outlet filter to aid in containing components of each chamber and/or to separate components of each chamber from one another. Inlet filters and outlet filters may prevent particles (or other structures to which lipase or other enzymes may be immobilized) from exiting chambers within formula processing device 250. Additionally or alternatively, the filters may prevent foreign objects from entering chambers within formula processing device 250, source 202, and/or conduit 262. Inlet and/or outlet filers may be located within or outside formula processing device 250 and/or each chamber 252, 254, 256.

Hydrolysis chamber 252 may be configured to hydrolyze fats, e.g., triglycerides, in nutritional formula 210 as nutritional formula 210 passes through hydrolysis chamber 252. Hydrolysis chamber 252 may contain a plurality of particles or other structures on which lipase may be immobilized, e.g., via covalent or ionic binding or by absorption, for example. In some embodiments, lipase may be immobilized to one or more walls and/or a filter within hydrolysis chamber 252, may be immobilized to a solid phase matrix, or may be free (not immobilized) within hydrolysis chamber 252. As nutritional formula 210 flows through hydrolysis chamber 252 and the structures (if included) therein, the immobilized lipase hydrolyzes the fats and triglycerides in nutritional formula 210, including triglycerides having LC-PUFAs (if included), breaking them down into monoglycerides and free fatty acids. The nutritional formula 210 containing the hydrolyzed lipids may then flow out of hydrolysis chamber 252, while the lipase may remain within hydrolysis chamber 252, e.g., bound to the particles or other structures in hydrolysis chamber 252. Examples of fat hydrolysis devices are disclosed in U.S. Patent Application No. 15/291,530, filed October 12, 2016, and U.S. Patent Application No. 14/378,856, filed August 14, 2014, now U.S. Patent No. 9,668,942.

Particles in hydrolysis chamber 252 may be formed as substantially spherical beads. In other embodiments, particles may be randomly shaped or irregular particles, or may be elliptical, oblong, donut-shaped, a prism, polygonal, elongated, or any other suitable shape or shapes. Particles may have a smooth or a textured surface, and/or may be shaped to increase or decrease their surface area. They may be formed of individual particles, which may each have substantially the same shape and/or surface or may have two or more different shape and/or surface combinations. They may be formed of any suitable material, and lipase may be immobilized on the particles in any suitable manner, e.g., via adsorption, ionic binding, covalent binding, cross-linking, encapsulation, and/or entrapment. Lipases may be immobilized on or in particles found within the hydrolysis chamber 252 such that the lipases are in fluid contact with nutritional formula 210 as nutritional formula 210 flows through hydrolysis chamber 252.

In some embodiments, hydrolysis chamber 252 may be made entirely or partly of a clear plastic or glass so that elements within hydrolysis chamber 252 are visible to a user. In some instances, this may allow the user to ensure proper flow through hydrolysis chamber 252, for example, by visual inspection. In other embodiments, hydrolysis chamber 252 may be opaque or may be made of any suitable material.

Particles (or other structures on which lipase may be immobilized) may be located between an inlet filter and an outlet filter in or on hydrolysis chamber 252. Such filters may retain particles within hydrolysis chamber 252 as nutritional formula 210 flows through formula processing device 250. In some embodiments, pore openings in the filters may aid in the emulsification and breakdown of fats in nutritional formula 210 as nutritional formula 210 flows through formula processing device 250.

In some embodiments, lipase may be immobilized to non-particle structures, e.g., monolithic carriers, that do not necessarily require the use of an inlet and/or outlet filter. The carriers may remain in hydrolysis chamber 252-or may be a part of hydrolysis chamber 252-even without the use of a filter. For example, such carriers may be too large to fit through an inlet and/or an outlet of hydrolysis chamber 252, may be attached or otherwise tethered to hydrolysis chamber 252, and/or may be formed as a portion of hydrolysis chamber 252.

Lipase included in the systems and methods disclosed herein may cleave all three bonds in a triglyceride or may cleave two out of three bonds in a triglyceride, i.e., at the sn-1 and sn-3 positions, leaving an sn-2 monoglyceride. Exemplary lipases may be obtained from animals, plants, and from many natural or genetically engineered microorganisms. In some embodiments, the lipase may include one or more of, e.g., *Chromobacterium viscosum, Pseudomonas fluorescens, Burcholderia cepacia, Thermomyces lanuginosus, Candida rugosa, Pseudomonas cepacia, Bacillus subtilis,* or *Rhizopus oryzae* lipase, or any other suitable wild-type or recombinant lipase or combination thereof.

While particles are described herein, it is appreciated that lipase may be immobilized in hydrolysis chamber 252 in any suitable manner. For example, lipases may be immobilized or contained within structures located inside hydrolysis chamber 252, such as beads, rods, fibers, sheets, monoliths, projections extending from portions of hydrolysis chamber 252, or other suitable structures. In some embodiments, lipases may be immobilized on or contained within a wall of hydrolysis chamber 252, and/or may be immobilized on one or more filters included in formula processing device 250.

In some embodiments, hydrolysis chamber 252 may be a sterilized or sterilizable container. In some embodiments, hydrolysis chamber 252 may be pre-filled with particles and/or lipases. In some embodiments, hydrolysis chamber 252 may be provided in a closed, sealed configuration and may be opened shortly prior to installation within formula processing device 250. In some embodiments, a user (e.g., healthcare provider, patient, patient guardian, pharmacist, or other user) may insert and/or install hydrolysis chamber 252 into formula processing device 250 prior to use. In some embodiments, hydrolysis chamber 252 may be pre-filled. In other embodiments, a user may fill hydrolysis chamber 252 with the desired type and/or amount of particles and/or lipases. In some embodiments, hydrolysis chamber 252 may be removable and disposable after use, and/or configured to be cleaned, sterilized, and refilled with particles and/or lipases such that it may be reused. In other embodiments, hydrolysis chamber 252 may be permanently housed within formula processing device 250.

Phytosterol processing chamber 254 may be configured to either remove or convert phytosterols in nutritional formula 210.

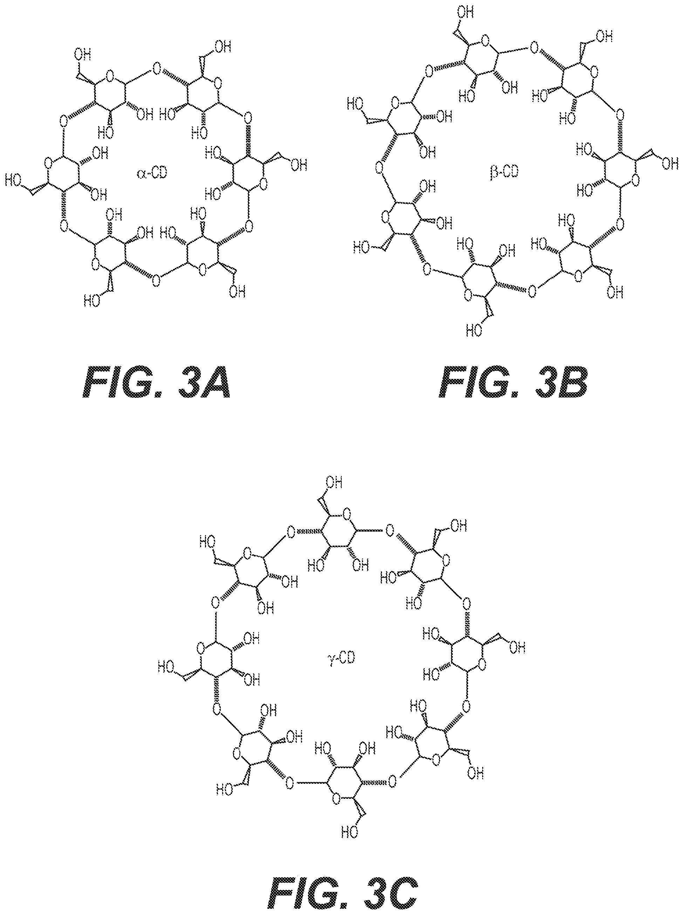

In embodiments in which phytosterol processing chamber 254 is configured to remove phytosterols, phytosterol processing chamber 254 may include one or more known sterol binding molecules to remove phytosterols from a nutritional formula. The sterol binding molecules may be part of a solid phase matrix. For example, phytosterol processing chamber 254 may include one or more sterol-binding molecules, such as cyclodextrins. Cyclodextrins (also referred to as cycloamyloses) are a family of compounds made up of sugar molecules bound together in ring structures (also known as cyclic oligosaccharides). Cyclodextrins may be produced, e.g., from starches, by enzymatic conversion. FIGS. 3A-3C depict the chemical structures of three common cyclodextrins-six-membered α-cyclodextrin (FIG. 3A), seven-membered β-cyclodextrin (FIG. 3B), and eight-membered γ-cyclodextrin (FIG. 3C). Both β-cyclodextrin and methyl-p-cyclodextrin (MβCD) remove cholesterol from cultured cells, with the methylated form MβCD being more efficient than β-cyclodextrin.

The water-soluble MβCD may form soluble inclusion complexes with cholesterol, thereby enhancing its solubility in aqueous solution. MβCD may be employed for the preparation of cholesterol-reduced products: the bulky and hydrophobic cholesterol molecule may become lodged inside cyclodextrin rings, which may then be removed. This mechanism (using MβCD or any other suitable cyclodextrin) may be employed to remove phytosterols within phytosterol processing chamber 254. In some embodiments, cyclodextrins may be present in phytosterol processing chamber 254 and then, upon passage of a volume of nutritional formula 210 into phytosterol processing chamber 254, phytosterol processing chamber 254 may be agitated, facilitating binding of cyclodextrins to phytosterols. Bound cyclodextrin-phytosterol complexes may be then removed by, for example, separating a supernatant of phytosterol-reduced nutritional formula 210 from the complexes, or allowing cyclodextrin-phytosterol complexes to be filtered into phytosterol collection reservoir 256. In other embodiments, cyclodextrin-phytosterol complexes may remain in phytosterol processing chamber 254 while the phytosterol-reduced nutritional formula 210 may be filtered out of phytosterol collection reservoir 256.

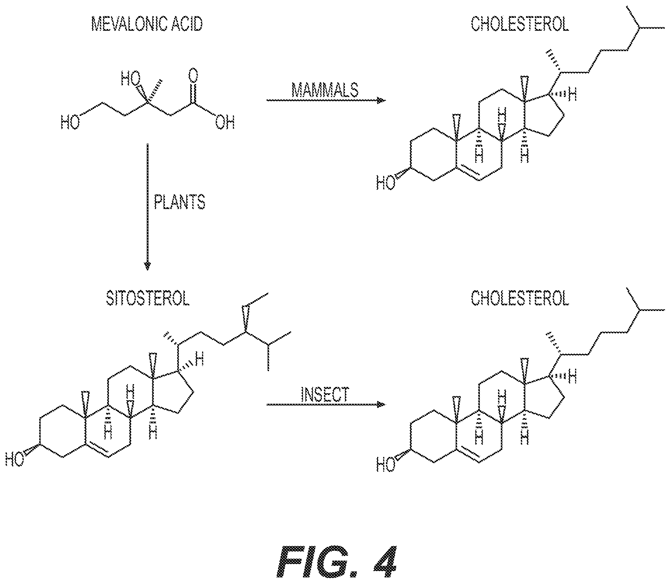

In embodiments in which phytosterol processing chamber 254 is configured to convert phytosterols into cholesterol e.g., using bioconversion pathways, phytosterol processing chamber 254 may include one or more enzymes known to bioconvert phytosterols into less toxic or non-toxic compounds that may be less likely to lead to the development of complications and/or side effects, e.g., hepatic complications. For example, phytosterol processing chamber 254 may include one or more enzymes to bioconvert phytosterols into cholesterols. Some insects and microbes may produce such enzymes. As in humans, cholesterol is a crucial structural component of cellular membranes in numerous microbes and insects. These organisms, however, lack the necessary metabolic pathways and corresponding enzymes to synthesize cholesterol from basic biological compounds, such as mevalonic acid. They do, however, possess enzymes that have the ability to take phytosterols commonly found in plants and convert them into cholesterol through enzymatic dealkylation of the C24, which is the critical carbon atom accounting for the structural differences between cholesterol and phytosterols. As depicted in FIG. 4, for example, while mammals are known to convert the key biological compound mevalonic acid into cholesterol, plants convert it into the phytosterol known as sitosterol. Insects may consume this sitosterol and convert it to cholesterol using particular enzymes. See, for example, W.H. Ling et al., Dietary phytosterols: a review of metabolism, benefits, and side effects, LIFE SCIENCES 57(3): 195-206 (1995).

Phytosterol processing chamber 254 may therefore include, e.g., enzymes bound to a solid matrix, particles, or other structures on which enzymes may be bound, and through which nutritional formula 210 may flow. The enzymes may metabolize phytosterols in nutritional formula 210 into cholesterol. In some embodiments, enzymes in phytosterol processing chamber 254 may not be bound and may be free-floating. The enzymes (immobilized or free) may be located along the flow path of nutritional formula 210 as it flows through phytosterol processing chamber 254. In such embodiments, a phytosterol collection reservoir 256 may not be necessary, as phytosterols may be metabolized instead of removed from nutritional formula 210. In other aspects, a collection reservoir 256 may be included in order to remove one or more metabolites from the nutritional formula and/or to remove un-metabolized phytosterols from the nutritional formula.

In some embodiments, phytosterol processing chamber 254 may include both cyclodextrins and phytosterol metabolizing agents, such that phytosterols are either metabolized into cholesterol, collected by cyclodextrins, or both. In this manner, if some phytosterol molecules are not bound to cyclodextrins, they may be converted to cholesterol, or if some phytosterol molecules are not converted to cholesterol, they are bound to cyclodextrins. In some embodiments, two phytosterol processing chambers 254 may be included, and, e.g., one chamber may metabolize phytosterols, and one chamber may remove phytosterols.

In some embodiments, phytosterol processing chamber 254 may include additional excipients. For example, citric acid, tocopherols, or other antioxidant excipients may be present in order to prevent or reduce oxidation of free fatty acids and monoglycerides present in nutritional formula 210 after nutritional formula 210 has passed through hydrolysis chamber 252.

In some embodiments, phytosterol processing chamber 254 may remove phytosterols using, e.g., adsorption, electrophoresis, electrostatic separation, extraction, field flow fractionation, ionic separation, filtration, centrifuging, gravimetrical separation, chromatography, crystallization, and/or using other techniques for removal of phytosterols, and/or using any of the techniques disclosed herein alone or in combination.

In some embodiments, phytosterol processing chamber 254 may be made entirely or partly of a clear plastic or glass so that elements within phytosterol processing chamber 254 are visible to a user. In some instances, this may allow the user to ensure proper flow through phytosterol processing chamber 254, for example, by visual inspection. In other embodiments, phytosterol processing chamber 254 may be opaque or may be made of any suitable material.

In some embodiments, phytosterol processing chamber 254 may be a sterilized or sterilizable container. In some embodiments, phytosterol processing chamber 254 may be pre-filled with phytosterol processing excipients (e.g., cyclodextrins, conversion enzymes, antioxidants, etc.). In some embodiments, phytosterol processing chamber 254 may be provided in a closed, sealed configuration and may be opened prior to installation within formula processing device 250. In some embodiments, a user (e.g., healthcare provider, patient, patient guardian, pharmacist, or other user) may insert and/or install phytosterol processing chamber 254 into formula processing device 250 prior to use. In some embodiments, phytosterol processing chamber 254 may be pre-filled. In other embodiments, a user may fill phytosterol processing chamber 254 with the desired type or amount of phytosterol processing excipients. In some embodiments, phytosterol processing chamber 254 may be removable and disposable after use, and/or configured to be cleaned, sterilized, and refilled with one or more phytosterol processing excipients such that phytosterol processing chamber 254 may be reused.

Phytosterol collection reservoir 256 may be a chamber configured to collect phytosterols removed from nutritional formula in, e.g., phytosterol processing chamber 254. Phytosterol collection reservoir 256 may be positioned so as to allow for efficient collection of phytosterols removed from nutritional formula 210. For example, in some embodiments, phytosterol collection reservoir 256 may be located below phytosterol processing chamber 254 in formula processing device 250. In other embodiments, phytosterol collection reservoir 256 may be located to the side of phytosterol processing chamber 254 or elsewhere. Phytosterol collection reservoir 256 may be directly connected to phytosterol processing chamber 254 or may be connected via one or more conduits 255.

In some embodiments, phytosterol collection reservoir 256 may be a sterilized or sterilizable container. In some embodiments, phytosterol collection reservoir 256 may be provided in a closed, sealed configuration and may be opened prior to installation within formula processing device 250. In some embodiments, a user (e.g., healthcare provider, patient, patient guardian, pharmacist, or other user) may insert and/or install phytosterol collection reservoir 256 into formula processing device 250 prior to use. In some embodiments, phytosterol collection reservoir 256 may be removable and disposable after use (e.g., after it has collected phytosterols), and/or configured to be emptied, cleaned, and sterilized such that it may be reused. A valve or other suitable flow control device may be included in one or more of phytosterol collection reservoir 256, phytosterol processing chamber 254, and/or conduit 255 to prevent or reduce backflow from phytosterol collection reservoir 256 to phytosterol processing chamber 254.

While formula processing device 250 is depicted in a configuration in which nutritional formula 210 may pass through hydrolysis chamber 252 prior to passing through phytosterol processing chamber 254, in alternative embodiments, the chambers may be arranged such that nutritional formula 210 may pass through phytosterol processing chamber 254 before passing through hydrolysis chamber 252. Alternatively, formula processing device may include only hydrolysis chamber 252, only phytosterol processing chamber 254, or a single chamber capable of performing the functions of both hydrolysis chamber 252 and phytosterol processing chamber 254.

Conduits 253, 255, 257 may be any type of conduits suitable for conveying nutritional formula 210 and/or portions thereof to and/or from chambers within formula processing device 250. Conduits 253, 255, 257 may be, for example, medical-grade tubing. Conduits 253, 255, 257 may connect adjacent chambers and/or to conduit 204 or connector 263 by any means known in the art, such as by a luer-lock connection, threads, projections, grooves, deformable or expandable structures, and/or any other suitable mechanism for connecting elements for carrying nutritional formula 210 and/or portions thereof. In some embodiments, one or more of conduits 253, 255, 257 may not be present and, for example, structures may be directly connected to one another. Additionally, one or more of chambers 252, 254, 256, and/or conduits 253, 255, 257 may include a valve or other suitable flow control device for promoting flow in a given direction and/or controlling the speed of flow through formula processing device 250.

Connector 263 may be any type of connector known in the art suitable for connecting conduit 257 in formula processing device 250 to conduit 262. Connector 263 may include, e.g., a luer-lock connection, threads, projections, grooves, deformable or expandable structures, and/or any other suitable mechanism. One suitable connector known in the art is the ENFit^{®} connector (GEDSA). In some embodiments, conduit 263 may not be present, and formula processing device 250 may connect directly to conduit 262 via any suitable connection means. In some embodiments, connector 263 may also include a valve or other fluid flow control mechanism.

Conduit 262 may be, for example, a feeding tube. In some embodiments, conduit 262 may be a parenteral feeding tube to feed nutritional formula 210 to the bloodstream of a subject through, for example, the external or internal jugular veins, the subclavian and axillary veins, the femoral vein, veins in the arms, veins in the legs, or veins in the scalp. Conduit 262 and system 200 may, in such embodiments, be used in keeping with standard parenteral feeding processes. In other embodiments, conduit 262 may be an enteral feeding tube, for example, a gastric, a nasogastric, a nasoduodenal, a nasojejunal, a gastrostomy, a gastrojejunostomy, a jejunostomy, a percutaneous endoscopic gastrostomy (PEG) tube, or a transjejunal feeding tube to feed nutritional formula 210 to the GI tract of a subject through, for example, the nose, mouth, stomach, or abdomen of the subject. In such cases, conduit 262 and system 200 may be used in line with standard enteral feeding practice. In other embodiments, conduit 262 may be a conduit to a receiving vessel, in which nutritional formula 210 may be collected after being processed by formula processing device 250.

Connector 265 may be configured to connect conduit 262 to needle 264 in a manner suitable for parenteral feeding, or to connect conduit 262 to other tubes or devices (not shown) for carrying nutritional formula from formula processing device 250 to a subject or a receiving vessel. Connector 265 may include, e.g., a luer-lock connection, threads, projections, grooves, deformable or expandable structures, and/or any other suitable mechanism. In some embodiments, connector 265 may also include a valve or other fluid flow control mechanism.

Needle 264 may be, for example, any needle suitable for providing a point of access for conduit 262 into a subject, in order to provide PN. Needle 264 may thus be any needle suitable for parenteral feeding known in the art. In some embodiments, for example, needle 264 may be a part of a cannula-over-needle device.

Use of system 200 and variations thereof may have several beneficial effects. For example, removal of phytosterols, or conversion of phytosterols to compounds that are not toxic or are less toxic, may decrease the likelihood of a subject developing PN-associated hepatic complications. Use of system 200 may, in some aspects, increase the amount of time that a subject may receive PN and/or increase the number of subjects eligible to receive PN. Further, the volume of PN formulation needed to increase fat absorption rates may be decreased, leading to more efficient absorption of critical fatty acids by the body, as well as other nutrients, such as, but not limited to, proteins and vitamins. Use of system 200 may also decrease the amount of time that PN is needed, since administering pre-hydrolyzed fats may lead to improved fat uptake and improved nutritional status in a shorter period of time, thereby allowing transition to enteral feedings or oral feedings sooner.

Additionally, use of system 200 may increase the number of total calories and/or energy obtained by a subject while keeping the volumes of nutritional formula provided to the subject (e.g., parenterally) relatively low due to the increased density of nutrients of the nutritional formula. For example, a larger volume of nutritional formula may need to be provided in order to obtain the same nutrient amount as a smaller volume of nutritional formula with hydrolyzed lipids.

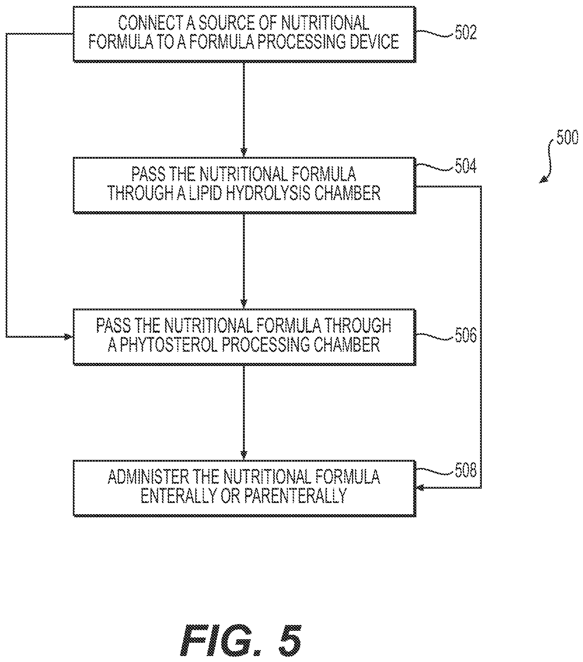

FIG. 5 depicts, in flow chart form, an exemplary method for preparing and administering a nutritional formula using, e.g., exemplary system 200. Those of ordinary skill in the art will recognize that one or more steps of the method depicted in FIG. 5 may be omitted or performed out of the order depicted in FIG. 5. Other steps may also be performed before, during, or after the steps depicted in FIG. 5.

According to step 502, a container of nutritional formula may be connected to a formula processing device 250. The container may be, for example, source 202, which may be connected to, e.g., formula processing device 250 via, for example, conduit 204. The nutritional formula may be, for example, nutritional formula 210. As has been previously described, the container may be connected to the formula processing device in any suitable manner known in the art.

Also as has been previously described, the nutritional formula may be prepared or primed in a variety of ways (e.g., mixed, agitated, heated, cooled, etc.) before or while being connected to the formula processing device.

Following step 502, the method may proceed to either step 504 or step 506. For example, the formula processing device may or may not include a lipid hydrolysis chamber.

According to step 504, the nutritional formula may be passed through a lipid hydrolysis chamber. The lipid hydrolysis chamber may be, for example, hydrolysis chamber 252. The nutritional formula may be passed through the lipid hydrolysis chamber (e.g., hydrolysis chamber 252) such that lipids in the nutritional formula (e.g., triglycerides) are hydrolyzed (e.g., into free fatty acids and monoglycerides). The nutritional formula may be passed through the lipid hydrolysis chamber in, e.g., any manner that has been previously described herein (e.g., using gravitational force, pressure differential, pumping or vacuum force, capillary action, etc.). These steps may be performed in any of the manners previously described herein, e.g., with respect to system 200.

Following step 504, process may proceed to either step 506 or step 508. For example, the formula processing device may or may not include a phytosterol processing chamber.

According to step 506, the nutritional formula may be passed through a phytosterol processing chamber. The phytosterol processing chamber may be, for example, phytosterol processing chamber 256. As the nutritional formula is passed through the phytosterol processing chamber, phytosterols in the nutritional formula may be either converted (e.g., bioconverted to cholesterols), or removed (e.g., bound to cyclodextrins and removed), or both. These steps may be performed in any of the manners previously described herein, e.g., with respect to system 200.

According to step 508, the nutritional formula may be administered parenterally, enterally, or orally. For example, the nutritional formula (e.g., nutritional formula 210) may be passed through a conduit (e.g., conduit 262) to a suitable attachment (e.g., needle 264) for parenteral administration to a subject. In other embodiments, the attachment may be a port, tube, or other device suitable for enteral feeding. In other embodiments, the attachment may be to a bottle, nipple, or other device suitable for oral feeding. In additional embodiments, the nutritional formula may not be administered, and may instead be stored in, e.g., a bottle, vial, beaker, tube, bag, or other container.

In some embodiments, step 508 may be delayed, for example, if the formula processing device is used to prepare a bulk nutritional formula, which is then later administered to subjects.

While step 504 is depicted as occurring before step 506 (in embodiments in which both steps are performed), it is contemplated that step 506 may, in some embodiments, precede step 504. It is also contemplated that steps 504 and 506 may be performed at the same time (e.g., in embodiments in which the formula processing device includes a chamber containing both lipase and a phytosterol processing excipient).

While many of the embodiments described above relate to devices, including point-of-care devices for the removal of phytosterols, it is also contemplated that phytosterols may be removed from bulk nutritional formula prior to storage of the nutritional formula to be administered to a subject at a later time. Removal of phytosterols according to such embodiments may occur with or without the hydrolysis of fats contained within the nutritional formula.

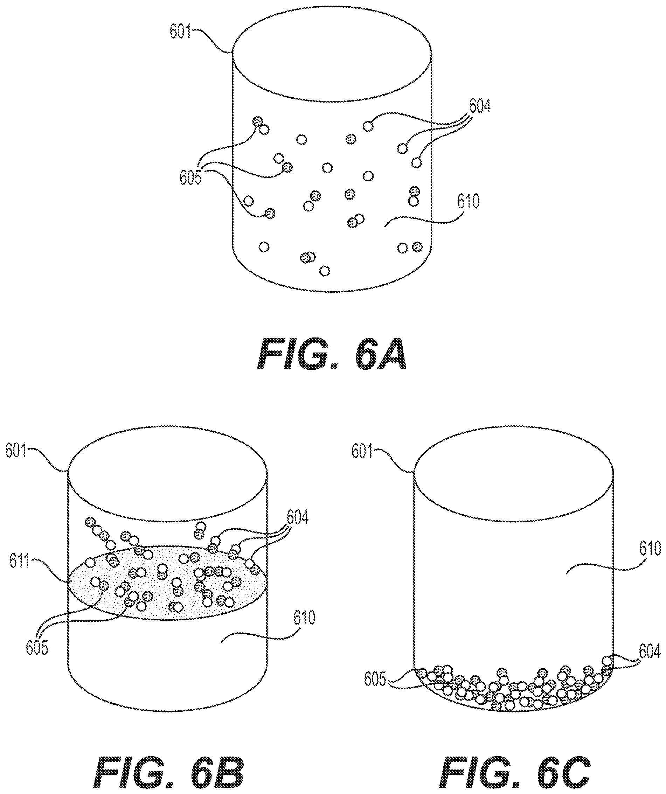

For example, a container may contain a bulk supply of nutritional formula. FIG. 6A depicts an exemplary bulk mixing tank 601 containing nutritional formula 610. Free (i.e., unbound) cyclodextrins 604 or cyclodextrins immobilized to an inert, insoluble matrix may be added to nutritional formula 610 in bulk mixing tank 601, or nutritional formula 610 may be added to cyclodextrins 604 already present in bulk mixing tank 601. For example, free and/or unbound cyclodextrins 604 may be added directly to a bulk supply of IVFE nutritional formula prior to administration of the nutritional formula to a person. For another example, free and/or unbound cyclodextrins 604 may be added directly to a bulk supply of IVFE nutritional formula prior to hydrolysis of the nutritional formula using a device like those according to the present disclosure, and/or after the bulk nutritional formula has been hydrolyzed by a device as disclosed herein. Cyclodextrins 604 may be added to bulk nutritional formula 610 in order to remove phytosterols 605 within nutritional formula 610 in tank 601 without passing nutritional formula 610 through a device (e.g., device 250 described above).

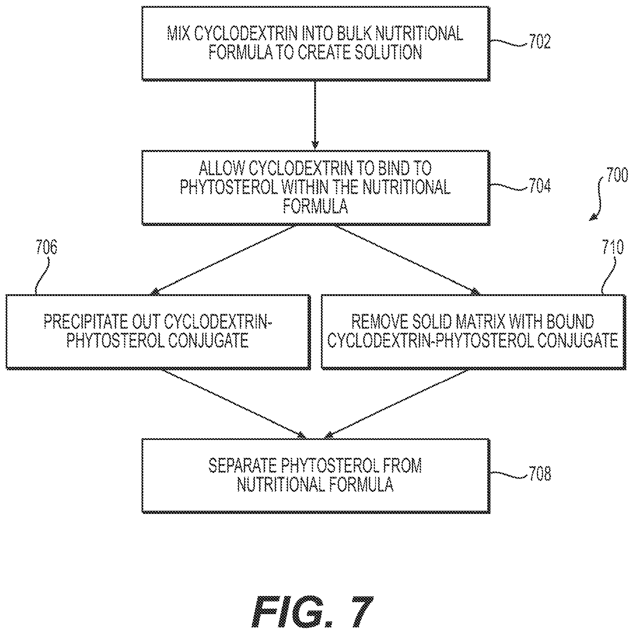

In an exemplary bulk processing method 700, the steps of which are depicted in FIG. 7, and the components of which are schematically depicted in FIG. 6A, nutritional formula 610 containing phytosterols 605 may be mixed with unbound cyclodextrins 604 (step 702 of FIG. 7). The mixture may be heated (for example, to 0 to 100°C, e.g., 20°C to 80°C, 40 to 60°C, or to 50 to 55°C), and/or agitated to promote dissolving of cyclodextrins 604 into nutritional formula 610 to create a solution of supersaturated cyclodextrins. The solution may be agitated to mix cyclodextrins 604 with nutritional formula 610 to facilitate binding of the free phytosterols 605 within nutritional formula 610 with cyclodextrins 604 (step 704 of FIG. 7). Heating and/or agitation of the solution may be carried out simultaneously or sequentially for, e.g., up to thirty minutes, up to an hour, up to five hours, or overnight (e.g., seven to ten hours or six to twelve hours) or more.

Following heating and/or agitation, the solution of cyclodextrins 604 and nutritional formula 610 may be cooled (for example, to 100°C to 0°C, e.g., 80°C to 20°C, or to 60°C to 20°C), allowing cyclodextrin-phytosterol conjugate to precipitate out of the supersaturated solution (step 706 of FIG. 7). Cooling may be achieved actively (e.g., by refrigeration) or passively (e.g., by allowing the solution to come to room temperature). This may facilitate removal of the phytosterol and/or the cyclodextrin to which the phytosterol is bound (step 708 of FIG. 7). For example, the precipitated phytosterol conjugate may be removed via a suitable separation method, e.g., centrifugation, filtration, elutriation, and/or adsorption to a suitable adsorbent (e.g., a polymeric resin). FIG. 6B depicts use of a filter 611 to separate the cyclodextrin-phytosterol conjugate from nutritional formula 610, and FIG. 6C depicts the cyclodextrin-phytosterol conjugate separated out from nutritional formula 610 after centrifugation.

In some embodiments, cyclodextrins 604 may be immobilized on a solid matrix, e.g., one or more insoluble polymer beads, rods, filters, screens, sheets, or other suitable insoluble structures. The immobilized cyclodextrins 604 and structures to which they are bound may be added to a bulk amount of nutritional formula 610 (step 702 of FIG. 7). The mixture of immobilized cyclodextrins 604 and nutritional formula 610 may be heated (for example, to 0°C to 100°C, e.g., 20°C to 50°C) and/or agitated to promote dissolving of cyclodextrins 604 to create a solution of supersaturated cyclodextrins. The solution may be agitated to mix cyclodextrins 604 with nutritional formula 610 to facilitate binding of the free phytosterols 605 within nutritional formula 610 with cyclodextrins 604 (step 704 of FIG. 7).

Alternatively, no supersaturated solution may be created, and heating and/or agitation may be carried out to promote binding of cyclodextrins 604 with phytosterols 605 while cyclodextrins 604 remain immobilized on insoluble matrices (rather than dissolved into solution). Heating and/or agitation may be carried out simultaneously or sequentially for, e.g., up to thirty minutes, up to an hour, up to five hours, or overnight (e.g., seven to ten hours or six to twelve hours) or more. In some embodiments, phytosterols 605 may bind to cyclodextrins 604 so that both phytosterols 605 and cyclodextrins 604 become immobilized on the solid matrix (or matrices), while in some embodiments, cyclodextrins 604 may unbind from the solid matrix (or matrices) when they bind with phytosterols 605, or a combination thereof (step 704 of FIG. 7).

In some embodiments, following heating and agitation, the mixture may be cooled (for example, to 100 to 0°C, e.g., 80 to 20°C or to 50 to 20°C). If cyclodextrins 604 separated from the solid matrix (or matrices) to which they were immobilized when binding with phytosterols 605, cooling may allow the cyclodextrin-phytosterol conjugate to precipitate out of solution (step 706 of FIG. 7). Cooling may be achieved actively (e.g., by refrigeration) or passively (e.g., by allowing the solution to come to room temperature). This may facilitate removal of phytosterol 605 and/or cyclodextrin 604 to which phytosterol 605 is bound. In some aspects, e.g., if cyclodextrin 604 remains immobilized on the solid matrix (or matrices) when binding with phytosterols 605, then, removal of the solid matrix may allow for removal of cyclodextrin 604 and phytosterol 605 from nutritional formula 610 (step 710 of FIG. 7), and cooling may or may not be performed. Removal of the immobilized and/or free cyclodextrin-phytosterol conjugate may be achieved via a suitable separation method, e.g., centrifugation, filtration, elutriation and/or removal of the solid matrix. Accordingly, the exemplary methods described above may allow for bulk removal of phytosterols from nutritional formulas using cyclodextrins.

FIG. 7 depicts, in flow chart form, an exemplary method for preparing bulk nutritional formula. Those of ordinary skill in the art will recognize that one or more steps of the method depicted in FIG. 7 may be omitted or performed out of the order depicted in FIG. 7. Other steps may also be performed before, during, or after the steps depicted in FIG. 7. Additionally some steps may be performed alternatively to one another (e.g., steps 706 and 710), alternative steps 706 and 710 may both be performed (e.g., if some cyclodextrin remains immobilized when binding to phytosterol while some cyclodextrin becomes free when binding to phytosterol), and/or any steps of FIG. 7 may be repeated multiple times. In some aspects, step 708 may be omitted if step 710 removes a sufficient amount of phytosterol from the nutritional formula.

In some aspects, one or both of the phytosterol or cyclodextrin separated from the bulk nutritional formula and/or the nutritional formula administered to a subject may be reused. For example, removed cyclodextrin may be reused subsequently to remove phytosterol from new batches of nutritional formula. In some aspects, removed phytosterol may be used in other food products, e.g., to replace cholesterol in margarine and/or dairy products. In some aspects, removed phytosterol may be reused as a nutritional ingredient by itself or in combination with one or more medicaments.

Following bulk removal of phytosterol, the nutritional formula may not be administered to a subject, and, may instead be stored in, e.g., a bottle, vial, beaker, tube, bag, or other container. The exemplary methods described herein may be used separate from device 250 described in relation to FIG. 2 or may be used prior to use of device 250. Bulk nutritional formulas having a first pass of phytosterols removed according to the method of FIG. 7 may or may not be passed through device 250 prior to administration to a patient.

## Claims

1. A device comprising:
one or more chambers;
immobilized lipase contained within one of the one or more chambers;
a phytosterol processing excipient contained within one of the one or more chambers;
an inlet fluidly connected to one of the one or more chambers, wherein the inlet is configured to receive nutritional formula into one of the one or more chambers; and
an outlet through which nutritional formula is configured to flow after passing through the one or more chambers.

2. The device of claim 1, wherein the one or more chambers comprises at least two chambers, and wherein the at least two chambers are fluidly connected to one another.

3. The device of claim 2, wherein the immobilized lipase is contained within a first of the at least two chambers, and the phytosterol processing excipient is contained within a second of the at least two chambers.

4. The device of claim 3, further comprising a third chamber fluidly connected to the second chamber.

5. The device of claim 1, wherein the phytosterol processing excipient is one of a cyclodextrin or a phytosterol metabolizing enzyme.

6. The device of claim 1, wherein the one or more chambers comprises one chamber, and the immobilized lipase and the phytosterol processing excipient are both contained within the one chamber.

7. The device of claim 1, wherein the one or more chambers comprises a first chamber and a second chamber, and the immobilized lipase and the phytosterol processing excipient are both contained within the first chamber, and the second chamber is a phytosterol collection reservoir.

8. The device of claim 1, further comprising a connector coupled to the outlet, wherein the connector is configured to connect the outlet to one of a parenteral feeding attachment or an enteral feeding attachment.

## Patentansprüche

1. Vorrichtung, aufweisend:
eine oder mehrere Kammern;
immobilisierte Lipase, die in einer der einen oder mehreren Kammern enthalten ist;
ein Hilfsstoff zur Phytosterolverarbeitung, der in einer der einen oder mehreren Kammern enthalten ist;
einen Einlass, der mit einer der einen oder mehreren Kammern fluidverbunden ist, wobei der Einlass zum Aufnehmen von Nährstoffrezeptur in eine der einen oder mehreren Kammern konfiguriert ist; und
einen Auslass, durch den Nährstoffrezeptur nach Durchlaufen der einen oder mehreren Kammern zu fließen konfiguriert ist.

2. Vorrichtung nach Anspruch 1, wobei die eine oder mehreren Kammern mindestens zwei Kammern aufweisen und wobei die mindestens zwei Kammern miteinander fluidverbunden sind.

3. Vorrichtung nach Anspruch 2, wobei die immobilisierte Lipase in einer ersten der mindestens zwei Kammern enthalten ist und der Hilfsstoff zur Phytosterolverarbeitung in einer zweiten der mindestens zwei Kammern enthalten ist.

4. Vorrichtung nach Anspruch 3, ferner aufweisend eine dritte Kammer, die mit der zweiten Kammer fluidverbunden ist.

5. Vorrichtung nach Anspruch 1, wobei der Hilfsstoff zur Phytosterolverarbeitung entweder ein Cyclodextrin oder ein Phytosterol metabolisierendes Enzym ist.

6. Vorrichtung nach Anspruch 1, wobei die eine oder die mehreren Kammern eine Kammer aufweisen und die immobilisierte Lipase und der Hilfsstoff zur Phytosterolverarbeitung beide in der einen Kammer enthalten sind.

7. Vorrichtung nach Anspruch 1, wobei die eine oder die mehreren Kammern eine erste Kammer und eine zweite Kammer aufweisen und die immobilisierte Lipase und der Hilfsstoff zur Phytosterolverarbeitung beide in der ersten Kammer enthalten sind und die zweite Kammer ein Phytosterolsammelbehälter ist.

8. Vorrichtung nach Anspruch 1, ferner aufweisend ein mit dem Auslass verbundenes Verbindungsstück, das zum Verbinden des Auslasses mit einem Gerät zur parenteralen Ernährung oder einem Gerät zur enteralen Ernährung konfiguriert ist.

## Revendications

1. Dispositif comprenant :
une ou plusieurs chambres ;
une lipase immobilisée contenue à l'intérieur d'une de la une ou plusieurs chambres ;
un excipient de préparation de phytostérol contenu à l'intérieur d'une de la une ou plusieurs chambres ;
une entrée connectée de manière fluide à une de la une ou plusieurs chambres, dans lequel l'entrée est configurée pour recevoir une formule nutritionnelle dans une de la une ou plusieurs chambres ; et
une sortie à travers laquelle une formule nutritionnelle est configurée pour s'écouler après passage à travers la une ou plusieurs chambres.

2. Dispositif selon la revendication 1, dans lequel la une ou plusieurs chambres comprend au moins deux chambres, et dans lequel les au moins deux chambres sont connectées de manière fluide l'une à l'autre.

3. Dispositif selon la revendication 2, dans lequel la lipase immobilisée est contenue à l'intérieur d'une première des au moins deux chambres, et l'excipient de préparation de phytostérol est contenu à l'intérieur d'une seconde des au moins deux chambres.

4. Dispositif selon la revendication 3, comprenant de plus une troisième chambre connectée de manière fluide à la seconde chambre.

5. Dispositif selon la revendication 1, dans lequel l'excipient de préparation de phytostérol est un d'une cyclodextrine ou d'une enzyme de métabolisation de phytostérol.

6. Dispositif selon la revendication 1, dans lequel la une ou plusieurs chambres comprend une chambre, et la lipase immobilisée et l'excipient de préparation de phytostérol sont tous deux contenus à l'intérieur de la une chambre.

7. Dispositif selon la revendication 1, dans lequel la une ou plusieurs chambres comprend une première chambre et une seconde chambre, et la lipase immobilisée et l'excipient de préparation de phytostérol sont tous les deux contenus à l'intérieur de la première chambre, et la seconde chambre est un réservoir de recueil de phytostérol.

8. Dispositif selon la revendication 1, comprenant de plus un connecteur couplé à la sortie, dans lequel le connecteur est configuré pour connecter la sortie à une d'une fixation d'alimentation parentérale ou d'une fixation d'alimentation entérale.
